# EUROPEAN PATENT SPECIFICATION

(11) **EP 0 563 131 B1**
(45) Date of publication and mention of the grant of the patent: **11.09.1996**
(21) Application number: 92901226.8
(22) Date of filing: 17.12.1991
(51) Int. Cl.: A61M 15/00

(54) **CLOSURE SYSTEM FOR INHALERS**
VERSCHLUSSSYSTEM FÜR INHALATIONSGERÄT
SYSTEME DE FERMETURE POUR INHALATEURS

(30) Priority: 17.12.1990 GB 9027255
(43) Date of publication of application: 06.10.1993
(73) Proprietor: MINNESOTA MINING AND MANUFACTURING COMPANY, St. Paul, Minnesota 55133-3427 (US)
(72) Inventor: ROBERTSON, Paul Andrew, Cambridge CB2 4QL (GB); BAUM, Eric Arthur, Loughborough, Leicestershire LE11 3LA (GB)
(74) Representative: Bowman, Paul Alan
(86) International application number: GB9102252
(87) International publication number: WO9211051

(56) References cited:
- WO-A-90/13327
- DE-A- 3 639 836
- GB-A- 2 074 454
- GB-A- 2 102 295
- US-A- 2 587 215
- US-A- 4 094 317
- US-A- 4 834 083

## Description

This invention relates to inhalers for the delivery of therapeutic substances to the respiratory system of a patient and in particular to a closure system for such inhalers to prevent the ingress of contaminants and/or air.

Since the metered dose pressurised inhaler was introduced in the mid-1950's, inhalation has become the most widely used route for delivering bronchodilators, offering a rapid onset of action and a low instance of systemic side effects. More recently, inhalation from a pressurised inhaler has been a route selected for the administration of other drugs, e.g., ergotamine, which are not primarily concerned with the treatment of a bronchial malady.

The metered dose inhaler is dependent upon the propulsive force of a propellant system used in its manufacture. The propellant generally comprises a mixture of liquified chlorofluorocarbons (CFC's) which are selected to provide the desired vapour pressure and stability of the formulation. Propellants 11, 12 and 114 are the most widely used propellants in aerosol formulations for inhalation administration.

Metered dose inhalers generally comprise a pressurised aerosol vial equipped with a metering valve positioned within an adaptor which shrouds the vial and defines a mouthpiece through which the patient inhales. It is necessary for the patient to inhale through the mouthpiece and to simultaneously depress the vial to fire the valve in order to administer the medicament. More sophisticated devices employing pressurised aerosol vials which automatically fire the valve in response to detection of patient inspiration through the mouthpiece are disclosed in European Patent No. 147028.

A further type of inhaler which dispenses doses of liquid medicament in the form of atomised droplets is disclosed in WO 92/11050 (published after the filing date of the present application). That Application discloses an inhaler device for dispensing droplets of liquid medicament to a patient comprising a body having a mouth piece or nasal adaptor, and a reservoir of liquid medicament in communication with an aerosol generator, the aerosol generator comprising a chamber for liquid medicament and a nozzle arrangement comprising a plurality of orifices in fluid flow relationship with liquid medicament in said chamber, means for cyclically pressurising the liquid medicament in said chamber such that liquid from said chamber is periodically expelled through the orifices as atomised droplets of liquid medicament so they may be inhaled via the mouth piece or nasal adaptor, the inhaler additionally comprising dosage control means for deactivating the aerosol generator after a predetermined time or a predetermined volume of liquid medicament has been expelled from the chamber.

The inhaler is capable of dispensing accurate doses of liquid medicament in the form of atomised droplets of a size suitable for inhalation therapy. The inhaler may be constructed in the form of a small battery powered, portable device, e.g., pocket sized, capable of being used to dispense metered doses of liquid drugs, as a replacement for conventional pressurised aerosol inhalers. In a preferred embodiment the inhaler comprises means to detect a patient's inspiration associated with a triggering means in order that the inhaler may be automatically triggered at the correct point of a patient's breathing cycle thereby avoiding the need for the patient to co-ordinate inspiration with operation of the inhaler.

The aerosol generator used in the inhaler comprises a chamber for liquid medicament and a nozzle arrangement comprising a plurality of orifices in fluid flow relationship with the liquid in the chamber. The orifices typically have a maximum opening in the range 2 to 50µm (microns) and produce atomised droplets having a size comparable to the diameter. For medicament intended to reach the alveoli of the lungs, the apertures desirably have a maximum opening of from 2 to 10µm (microns), preferably below 5µm (microns), in order to produce atomised droplets comparable to that range. For liquid medicament intended to be administered to the nasal passage, mouth, throat or other parts of the respiratory system, the larger orifices may be employed. The orifices may have the same or different diameters. Preferably, the orifices are tapered towards the intended outlet for the liquid. The orifices are generally spaced from each other by distances within the range 20 to 200 microns. The nozzles may be fabricated by the same technique used to manufacture microsieves, e.g., electro forming in nickel. Alternatively, the nozzle arrangement may be formed by patterned anisotropic etching through a thin semiconductor wafer, e.g., of silicon or germanium. Alternatively, plastics nozzle arrays may be used.

The thickness of the nozzle arrangement is typically in the range 20 to 100µm (microns).

A preferred nozzle array comprises an electroformed nickel foil about 10µm thick with holes approximately 6µm in diameter set on a 50µm pitch. A reinforcing grid of about 60µm thickness is electroformed over the thinner foil for additional strength. Such foils are commercially available from Stork-Veco BV of Holland and have been sold for use as microsieves.

The aerosol generator is constructed to cyclically pressurise liquid in the chamber causing the liquid periodically to be expelled through the orifices as atomised droplets of liquid. The cyclic pressurisation may be achieved utilising a piezo-electric element which is caused to vibrate ultrasonically and acts directly or indirectly on the liquid.

In one embodiment the chamber of the aerosol generator comprises a flexible disc forming at least part of a wall of the chamber, the flexible disc being attached to a piezo-electric element, to form a vibrator element. The vibrator element is excited by a suitable resonant frequency generally in the ultrasonic range of 10 to 500 kHz, typically 50 to 250 kHz. Ultrasonic pressure waves propagate through the disc, cavity walls and liquid, resulting in liquid being forced periodically at ultrasonic frequencies through the nozzles. The use of a resonant mode above the fundamental mode frequency enables low drive voltages and power to achieve high liquid ejection flow rates through the nozzle arrangement. The flexible vibrator element may conveniently be positioned in a wall opposite to the nozzle arrangement, although this configuration is not essential and the vibrator element may be in any position which will propagate a pressure wave within the liquid causing droplets to be expelled through the nozzle arrangement.

Suitable vibrator elements are commercially available from Kyocera and Murata of Japan and have been sold for use as piezo acoustic buzzer elements. The elements are brass 20mm in diameter bonded to a 14mm diameter piezo electric disc. The brass disc may be polished and electroplated with nickel to give a corrosion resistant finish.

The material forming the remaining walls of the cavity of the aerosol generator has been found to give best results if it is made from a relatively low acoustic loss and impedance material. For example, aluminium alloy, Perspex, Polycarbonate and ABS plastic have been found to work well, whereas nickel and stainless steel are not so effective.

One advantage of ABS is that it may be injection moulded to form a complete assembly. In this case the aerosol generator disc may be linked to the body of the device by a 'limb' which contains the liquid feed channel.

The adhesive bonding between the vibrator element and the cavity walls is also important. Two part epoxy resins, e.g., Araldite commercially available from Ciba-Geigy in the United Kingdom, work well whereas silicone rubber does not, suggesting that good acoustic coupling between the components is desirable. The nozzle array may also be conveniently bonded with epoxy resin. Hot melt adhesives may also be employed.

In an alternative embodiment of the invention the nozzle assembly is vibrated. The nozzle assembly may be flexible and comprises a piezo-electric element, e.g., in the form of a ring attached to the nozzle array extended around the orifices such that when the piezo-electric element is excited it causes vibration of the nozzle arrangement at ultrasonic frequencies resulting in cyclic pressurisation of the liquid in the chamber and ejection of droplets of liquid through the orifices.

In a preferred embodiment the nozzle assembly is vibrated by a vibrator element comprising a piezo-electric ring secured to a metal disc of larger diameter, the vibrating element having a central aperture through which droplets from the nozzle array are emitted. The vibrating element is preferably secured only over its central portion, either directly to the nozzle array or to the housing of the chamber in close proximity to the nozzle array e.g. over a central portion of about 4mm diameter, such that ultrasonic energy is transferred directly to the nozzle array. This arrangement allows the outer area of the vibrating element, which is typically about 20mm diameter, to vibrate freely as a resonator and enables aerosol generation to occur with an input power to the piezo-electric element of about 0.5W. Also the arrangement has less tendency to draw tiny air bubbles in through the nozzles during operation since this reduces the tendency for and effects of, vibrational mode hopping which can occur if the piezo drive is attached around its periphery.

The drive frequency for this arrangement is still typically in the range 250 to 400kHz where the vibrating element operates in an overtone mode with a complex mode pattern. It is likely that this frequency corresponds to the radial mode of the piezo which in turn excites other modes in the metal element. The use of overtone frequencies of the metal element i.e. those above the fundamental allows thin, low cost pieces of piezo to be employed. Generally, the thickness of the piezo element and the metal disc should be similar. Hence if the metal thickness were increased to raise the fundamental resonant frequency of the vibrating element a thicker piezo element, and theefore of higher cost, would also be required.

The overall dimension of the aerosol generator may be small, e.g., 20 millimetres in diameter and 3 millimetres thick and is capable of delivering volumes of several microlitres of atomised droplets of liquid in a time period of about 0.5 seconds.

The chamber of the aerosol generator is supplied with liquid medicament from a reservoir. It has been found that the presence of air in the chamber or liquid, even in the form of minute bubbles may deleteriously affect the performance of the aerosol generator, particularly if air bubbles are present in the region of the nozzle arrangement. This effect is less marked with the arrangement where the vibrator element is attached directly to the nozzle array or to the housing in close proximity to the nozzle array. The reservoir may be arranged to supply liquid to the chamber only when the inhaler is used, although in practice it has been found that it is difficult to repeatedly fill and empty the chamber of the aerosol generator without entrapping air bubbles during the filling. Accordingly, it is preferred that the chamber is permanently filled with liquid. In order to avoid the problem of liquid leaking from the chamber via the nozzle arrangement during the time when the inhaler is not in use, e.g., storage in a pocket, the nozzle arrangement may be conveniently sealed with a cap.

Dry powder inhalers in which the medicament is introduced into the inhalation device from a capsule are disclosed in U.S. Patent Nos. 3,948,264, 3,971,377 and 4,147,166 and British Patent No. 1479283. Dry powder inhalers having a reservoir of dry powder from which unit doses are transferred to a chamber by means of a delivery system, such as a rotating perforated membrane in which the perforations are filled with powder from the reservoir, are disclosed in British Patent Application Nos. 2102295 and 2144997 and European Patent Application Nos. 69715, 79478 and 166294.

Co-pending International Patent Application No. PCT/US/90/02412 (publication No. WO-A- 9 013 328) discloses a dry powder inhalation device comprising a housing defining a chamber in communication with a patient port in the form of a mouthpiece or nasal adaptor, and an elongate carrier bearing a powdered medicament, the device being constructed and arranged such that areas of predetermined size of the elongate carrier may sequentially be exposed within the chamber, the device comprising one or more air inlets such that when a patient inhales through the patient port an air flow is established from the air inlet(s) to the patient port through the chamber such that particles of the powdered medicament of respirable size from said exposed area of the elongate carrier are entrained within the air flow.

The dry powder inhaler is capable of delivering multiple, uniform doses of a medicament to a patient. The device is simple to operate and does not require the patient to insert capsules of medicament or rely upon a separate reservoir of medicament in order to load the device for use. The medicament is generally preloaded on an elongate carrier, sections of which are sequentially exposed in the chamber for dispensing the medicament. The elongate carrier may be conveniently loaded on a spool (in a similar manner to a photographic film) or in a cassette (in a similar manner to an audio cassette). The elongate carrier may have any ratio of length : width but is generally greater than 5 : 1, usually greater than 10 : 1 preferably between 100 : 1 and 1000 : 1.

The preloaded elongate carrier can take a variety of forms, but preferably is a tape, web, belt or cord. The powdered medicament may be retained on the carrier by electrostatic attraction, van der Waals forces, physical attraction, mechanical binding, wedging or by a cover layer or an overlying layer of the same carrier when the carrier is wound etc. One or more surfaces of the carrier and optionally the interior of the carrier may be configured to assist in retaining the particles of powder.

The format of the carrier in the most preferred embodiment is a tape. The nature of the carrier dictates the method of transport between storage means and the chamber where aerosolisation takes place. In a preferred embodiment, storage of preloaded carrier is effected by winding on a spool which is contained within a cassette. Use of a tape web or belt allows other conformations to be imparted to the stored carrier by folding, for example, as a concertina conformation which has the advantage that the medicament bearing surfaces are in association and thereby prevent net transfer of medicament during storage. Each fold may define a unit dose of medicament. Folding along the longitudinal axis of the tape, referred to as hybrid folding, may also reduce unwanted net transfer of medicament. Cord or string may conveniently be stored as a coil.

The device includes means for advancing the elongate carrier through the chamber to sequentially expose areas of the carrier for release of medicament during inhalation by the patient. The means for advancement may take a variety of forms depending upon the type of elongate carrier and whether the exposed areas of carrier are to be retained within the device. For example, tapes webs and belts may include a series of apertures which are engaged by one or more sprocketed guide wheels or rollers in a similar manner to a camera or printer. Alternatively, or in addition, the carrier may be wound on a take-up spool, rotation of the spool directly or via a drive belt causing the carrier to advance. The device may also include means for tensioning or otherwise maintaining the exposed area of the carrier within the chamber during inhalation by the patient.

The elongate carrier may be advanced into the chamber prior to inhalation by the patient preferably or the carrier may be advanced into the aerosolisation chamber during inhalation to protect the powdered medicament from premature exposure. For example in one embodiment of the inhaler an unexposed area of carrier is rapidly advanced into the chamber upon actuation, and is rapidly decelerated or brought to an abrupt halt and preferably is impacted thereby imparting sufficient energy to the medicament particles to effect their displacement from the carrier into the air stream.

In a preferred embodiment the elongate carrier is stored in a cassette both before and after exposure. The cassette may comprise one or preferably two spools together with idlers or other rollers and include an exposure frame positioned within the chamber, through which the carrier is advanced. The cassette may be removable to allow the device to be recharged with a new cassette. However, it is not essential for the exposed areas of the carrier to be retained within the device and spent carrier may be advanced to the exterior of the device through a slot in the housing whereupon disposal may be effected by the patient, optionally with the aid of a cutting edge. This arrangement is particularly suitable for a tape carrier which has transverse perforations to facilitate tearing off spent carrier.

The device preferably additionally comprises means for releasing medicament of respirable size from the exposed area of carrier independent of the patients' inspiratory effort. The medicament release means overcomes the binding of the medicament particles to the carrier by mechanical effort e.g. impaction, vibrations, gas flow etc. or electrostatically.

The means for releasing medicament from the carrier during inhalation is preferably triggered in response to the patient inhaling in order to avoid the patient having to synchronise inhalation and actuation of the release mechanism. Airflow detection may conveniently be accomplished by means of a movable vane positioned within the chamber or patient port, motion of the vane causing actuation of the release mechanism. Such a vane may also be constructed to prevent a patient exhaling through the device and/or preventing exhaled air from reaching the stored carrier thereby avoiding any problems associated with moisture. Other such sealing means may also be employed. A suitable desiccant cartridge may be incorporated into the inhaler or may be incorporated into the carrier cassette.

The inhalation devices of the pressurised aerosol vial type, the liquid dispensing type and the dry powder type generally comprise a mouthpiece cover to protect the device from the ingress of contaminants, such as dirt etc., when the device is not in use, e.g., carried in a pocket or handbag etc. The mouthpiece cover may be in the form of a separate cap which is a push-fit over or into the mouthpiece or may be in the form of a hinged cover which can form part of a protective casing. Alternatively, the mouthpiece may be kept covered in a retracted position and may be moved into an extended position for use by manual rotation of the cover by the patient, as disclosed in British Patent Specification No. 2074,454. In each case closing of the cover requires a positive action by the patient.

British Patent No. 898649 discloses a dispenser comprising a mouthpiece with one or more inlet openings and valve means for controlling the injection of finely divided material in an air stream into said mouthpiece, which valve means is incapable of being opened solely by the pressure of said injection but can be opened by a pre-determined degree of suction caused by drawing in air by inhalation through said opening or openings or thereby permitted to be opened by said pressure. The finely divided material is introduced into the dispenser in a cartridge which is thereafter perforated and the air stream is generally established by a squeeze bulb. The patient is caused to make a strong inspiratory effort without at the same time filling his lungs prematurely with air and only when he is making a sufficiently strong effort to activate the valve means can he get both air and a dose of powder. The valve means is biased to the closed position.

WO 90/13327 discloses an inhalation device for dry powder comprising a housing defining a chamber for receiving a dose of powdered medicament in communication with a patient port in the form of a mouthpiece or nasal adapter which may comprise a cover, the inhalation device additionally comprising deagglomeration/aerosolisation means to deagglomerate and/or assist aerosolisation of said dose of powdered medicament, which means is operable by a patient-independent energy output source, detection means to detect patient inspiration through the patient port, and, control means to actuate said deaglomeration/aerosolisation means in response to detection of patient inspiration by said detection means.

The present invention provides a metered dose inhalation device which comprises a cover for the mouthpiece and/or a cover for an aerosol generator.

Therefore according to the present invention there is provided an inhalation device comprising a housing containing a medicament reservoir and defining a patient port in the form of a mouthpiece or nasal adaptor, means for metering a dose of medicament from said medicament reservoir and means to aerosolise said dose of medicament, the inhalation device additionally comprising a cover for the patient port or aerosolisation means movable between open and closed positions, the cover requiring an inspiration independent energy source for opening and having a self-closing action such that the patient port or aerosolisation means is sealed to protect the medicament whenever the inhalation device is released by the patient.

The invention provides an inhalation device having a cover which will automatically seal the mouthpiece or aerosolisation means when the device is released by the patient thereby ensuring the device is sealed against contaminants when not in use. The self-closing action of the cover may be achieved by any means, e.g., mechanical, electromechanical, pneumatic, hydraulic etc., and the self-closing action may be triggered at any desired time up to the release of the inhalation device by the patient. For example, the cover may be configured to close when the patient ceases to inspire through the mouthpiece after delivery of a dose of medicament, when a predetermined portion of the inhalation device, i.e., actuation means, e.g., button, switch, lever, slide, etc., is released by the patient or when the complete inhalation device is released by the patient. The self-closing action preferably has a fast response time such that the cover will be fully closed by the time the device has fallen l metre, more preferably, 0.5 metre under the influence of gravity. Thus, in the event of the patient dropping the inhalation device, the cover should be fully closed before the device impacts with the ground or other surface, e.g., a table.

The physical opening of the cover does not require inspiratory effort by the patient and is attained by mechanical or electromechanical effort. However, the device may include means to detect inspiration which is utilised to trigger the opening of the cover by an inspiration independent energy source.

The invention is applicable to all inhalation devices, including pressurised aerosol vial, liquid dispensing and dry powder type devices. In the case of dry powder inhalation devices and pressurised aerosol vial inhalation devices, the cover is generally present on the mouthpiece and seals the mouthpiece against the ingress of contaminants. In a simple form the cover may comprise a single shutter or a pair of shutters which are resiliently biased to the closed position and opened by the patient moving a lever or slide arrangement which when released causes the cover to close. In a more sophisticated inhalation device having a power source, e.g., a battery or compressed gas, the cover may be opened and closed from energy derived from the power source in response to a patient activating and deactivating a switch or valve.

Inhalation devices comprising an aerosol generator for generating atomised droplets of liquid by cyclically pressurising a liquid in a chamber causing the liquid to be expelled through orifices of a nozzle arrangement in the generator tend to be deleteriously affected by the presence of air bubbles in the generator, particularly in the vicinity of the nozzle arrangement. It has been found that if the nozzle arrangement is not sealed when the device is not in use there is a tendency for air to enter the aerosol generator through the nozzle arrangement, particularly if the inhalation device is subjected to jolting, jarring, percussion etc. In accordance with the invention such an inhalation device may comprise a cover which possesses a self-closing action to seal the aerosol generator against the ingress of air when the inhalation device is not in use. Such a cover also prevents the loss of liquid from the aerosol generator by evaporation. The self-closing action is preferably triggered when the patient ceases to inspire through the mouthpiece after delivery of a dose of medicament but may be triggered by the patient deactivating a switch or releasing the inhalation device. In addition to the cover for the aerosol generator the inhalation device may include a cover for the mouthpiece which may have a self-closing action as described above.

The invention will now be described with reference to the accompanying drawings in which:
Figures 1a to 1c represent an inhalation device in accordance with the present invention of the type incorporating a pressurised aerosol vial;
Figures 2a and 2b represent a cross-section and side view of an aerosol generator for generating atomised droplets of liquid suitable for use in an inhalation device of the invention;
Figures 3a and 3b represent a horizontal section and a fragmentary vertical section of an inhalation device in accordance with the invention incorporating an aerosol generator of the type disclosed in Figures 2a and 2b;
Figure 4 represents a cross-section through a further inhalation device in accordance with the invention incorporating an aerosol generator of the type disclosed in Figures 2a and 2b;
Figure 5 represents a schematic cross-section and block diagram of a further inhalation device in accordance with the invention incorporating an aerosol generator of the type disclosed in Figures 2a and 2b, and
Figure 6 represents a cross-section through a further aerosol generator suitable for use in an inhaler of the invention.

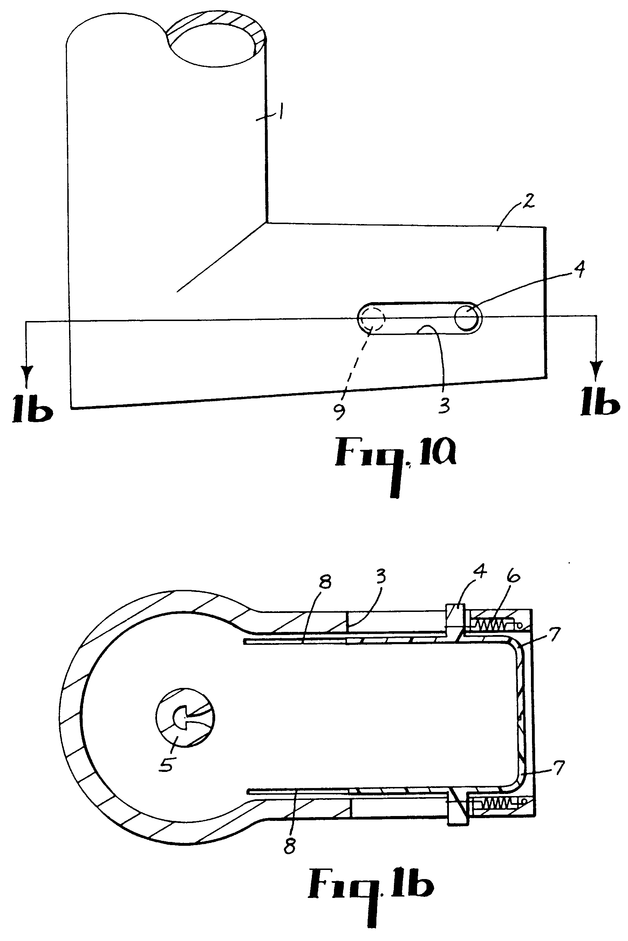

Referring to Figures 1a to 1c, the inhalation device comprises a housing (1) which contains an aerosol vial (not shown) and defines a mouthpiece (2). The valve (not shown) of the aerosol vial is located within nozzle block (5) (Figures 1b and 1c) and the device is operated by depressing the aerosol vial causing the valve to fire, dispensing the medicament through the nozzle block (5) towards the mouthpiece (2) whilst the patient inhales through the mouthpiece (2).

The mouthpiece (2) is generally sealed by a pair of flexible shutters (7) which are located within groove (8). The shutters (7) are attached to pins (4) which are located within slots (3) in the walls of the mouthpiece (2) and are biased to the closed position by means of springs (6).

Figures 1a and 1b show the inhalation device when released by the patient with the shutters in the closed position. In order to use the device the patient must slide the pins (4) along slots (3) to the position shown at (9) (Figure 1a) thereby opening the shutters as shown in Figure 1c. As soon as the patient releases the pins (4), after taking a dose of medicament or accidentally e.g., by dropping the device, the shutters automatically self-close under the influence of springs (6) thereby sealing the mouthpiece. Whilst this embodiment has been described with reference to an inhalation device comprising a pressurised aerosol vial it will be appreciated that the cover arrangement is equally applicable to dry powder inhalers or liquid dispensing inhalers.

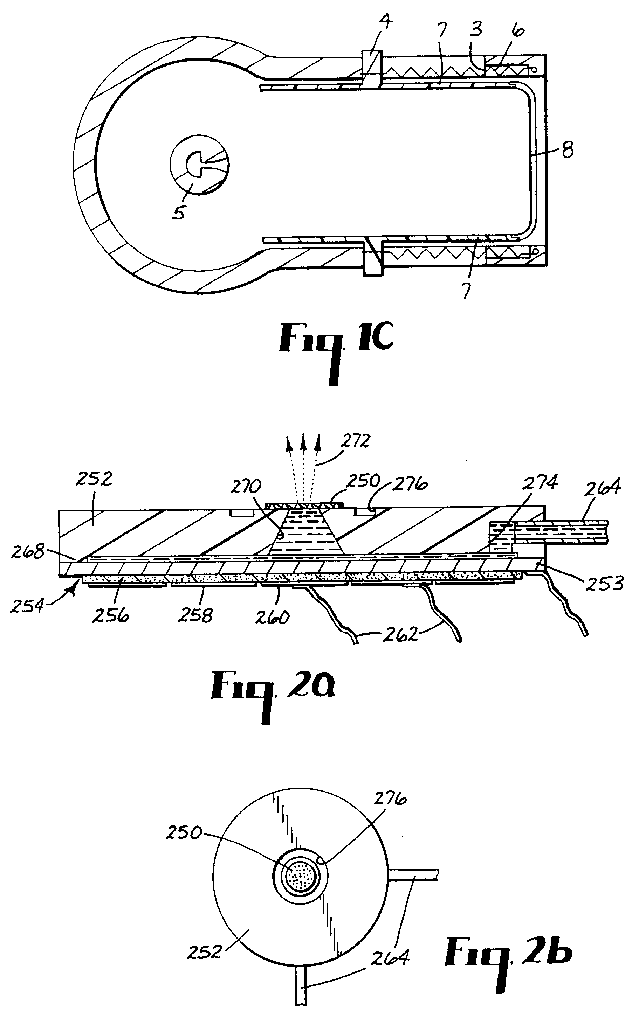

Figures 2a and 2b, illustrate an aerosol generator suitable for use in the invention which comprises a disc of material (252), such as aluminium alloy or plastics, e.g., Perspex (registered Trade Mark), formed by machining, moulding or other shaping process to produce a central conical or exponentially shaped port (270), a mounting rim (268), filling ports (274) and a recessed groove (276). A vibrator element (254), such as those manufactured by Kyocera and Murata for audio sounders, is attached to the disc (252) around the mounting rim (268) by adhesive or bonding techniques. The vibrator element (254) comprises a brass disc electrode (253) about 0.2mm thick and 20mm diameter onto which is bonded a smaller disc of piezo-electric material (256). One or more electrodes (258) and (260) are formed on the piezo-electric material (256) and lead wires (262) are connected to these electrodes and to disc electrode (253). When an electric field is applied between the electrodes, the vibrator element bends and may be excited into mechanical resonance by application of an alternating voltage of appropriate frequency. An array of nozzles (250) is attached over the narrow opening of the port (270) by adhesive or other bonding technique. The groove (276) prevents excessive spreading of adhesive over the disc surface where a cap may need to seal. The liquid to be ejected is introduced into the cavity formed by the disc (252), vibrator element (254) and nozzle array (250) by one or more feed tubes (264), sealed into the filling ports (274). When the vibrator element (254) is excited into a suitable resonance then ultrasonic vibrations are transferred into the liquid (216) and around the rim of the vibrator element into the disc (252) by motion of the vibrator element (254). These effects result in ultrasonic pressure pulses within the liquid (216) behind the nozzle array (250) and droplets (272) are formed as the liquid (216) is periodically ejected through the nozzle array (250) at ultrasonic frequencies.

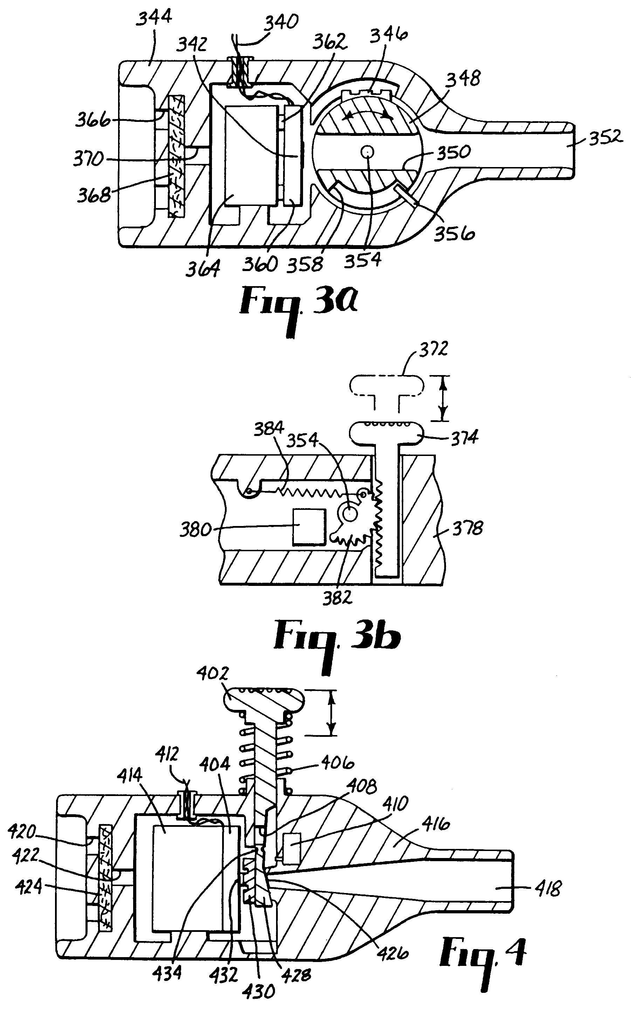

Figure 3a shows a cross-section diagram of a portable hand-held inhaler featuring an ultrasonic aerosol generator (360), such as of the type shown in Figures 2a and 2b and drug reservoir, flow valves and dose gauge (364) mounted within a housing (344). The aerosol generator is mounted on compliant mounts (362) and is supplied power via electrical leads (340) which pass through the housing (344). The air inlet to the inhaler is comprised of apertures (366), a filter (368) and an entrance port (370) into which (not shown) a flap valve and/or air flow sensor may be fitted if required. A rotating cylinder (348) close fitting inside the casing (344) and bearing an aperture (350) acts as a shut-off valve to air flow through the device when rotated anticlockwise 90° on axle (354), from the open position shown in Figure 3a. When open as shown, air is drawn through the device when a patient inhales through the mouthpiece (352) and droplets emitted from the aerosol generator (360) at position (342) are mixed with the indrawn air which flows around the components (360 and 364). A stop pin (356) runs in a slot (358) in the cylinder (348) to limit the rotational travel of the cylinder (348). When the cylinder (348) is rotated to the shut position, a compliant sealing cap (346) seals over the face of the aerosol generator (360). The cylinder is rotated during use by an arrangement such as that shown in Figure 3b which illustrates a mechanism which can be implemented on the side of the device shown in Figure 3a.

In Figure 3b a rack and pinion arrangement is shown which may be used to rotate the cylinder (348). A manually operated plunger (374) running in a housing (378) carries teeth (376) which mesh into the half pinion (382) which is mounted onto the cylinder axle (354). With the plunger (374) held down as shown, the axle and cylinder are rotated to the open position against the force of a tension spring (384). The full open position may be detected with a micro-switch (380) operated by the half pinion (382). If the plunger (374) is released then it springs up to position (372) and the cylinder (348) rotates to the shut position, sealing the aerosol generator (360) and closing the air path through the unit. Hence dust and other foreign matter cannot enter the unit during storage.

An alternative design of inhaler with self-closing action is shown, illustrated in cross-section, in Figure 4. In this case, the unit is shown almost fully closed and sealed. A plunger (402) runs in the casing (416) and is spring loaded by a compression spring (406) into the position shown. The aerosol generator (404), powered through leads (412), is sealed over its droplet emitting area (432) by a cap (430). The cap (430) is attached to a wedge (428) which is fixed to the plunger (402) via a flexure hinge (434). The force of the spring (406) pulls the wedge (428) against a sloping face (426) at the terminus of mouthpiece (418) such that the cap (430) is forced against she aerosol generator (404) thereby sealing it. When the plunger (402) is pushed down the force of the cap (430) against the aerosol generator area (432) is released and the cap and plunger wedge moved downwards. When the plunger (402) is fully depressed, a micro-switch (410) is activated and an aperture (408) in the plunger (402) aligns with the aerosol emitting area (432). When air is drawn in through the device by a patient inhaling through the mouthpiece (418), it travels through apertures (420), filter (424) and entrance port (422), which may contain a valve or air flow sensor. The in-drawn air flows round the reservoir (414) and aerosol generator (404) and is mixed with the aerosol droplets emitted from (432) as it flows through the aligned apertures through (402 and 426). If the plunger (402) is released then the unit seals shut under the force of the spring (406).

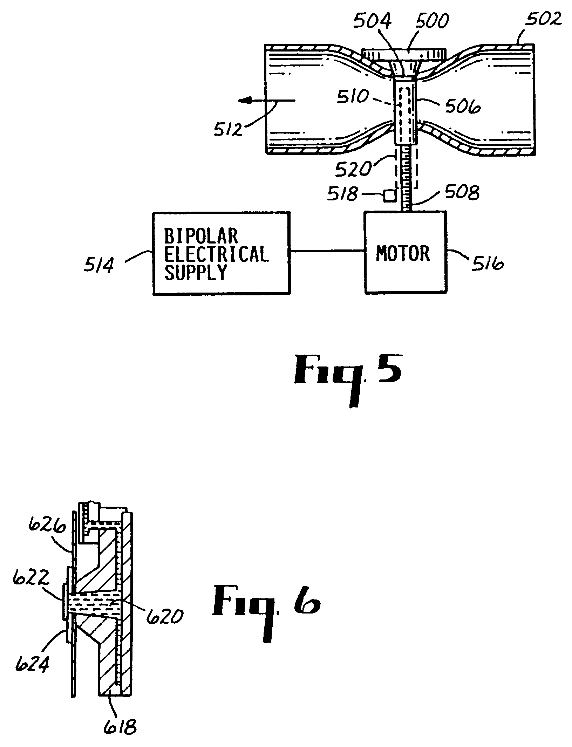

Figure 5 is a schematic diagram of an electromechanical closure system combined with a droplet air mixer venturi in an inhaler having an aerosol generator of the type shown in Figures 2a and 2b. The aerosol generator (500) emits droplets into the throat of a venturi (502). The air flow through the venturi (502) throat is at right angles to the droplet emission direction and thorough mixing occurs before the exit flow (512).

The nozzle cover (504) is attached to a carrier (506) which has an internal thread (510). The carrier (506) and cover (504) are moved linearly by a leadscrew (508), which matches the thread (510), and a motor (516). The motor (516) is driven by a bipolar electrical supply (514) which can reverse the motor direction to open or close the cover. When the cover is open the carrier rests in position (520) against a mechanical stop (518) with the cover flush with the venturi throat wall, allowing the air flow to be almost undisturbed. The motor may be controlled by a switch (not shown) which is biased to a position closing the cover and must be depressed by the patient to open the cover. Alternatively, the inhaler may have means to sense inspiration by the patient and provide a signal to actuate the motor opening the cover, the motor being reversed to close the cover upon dispensing a dose of medicament.

The aerosol generator is shown in Figure 6, comprises a housing (618) defining a chamber (620) having at one end a nozzle array (622). The chamber is in communication with a reservoir via a dosage gauge. A vibrator element comprising a piezo-electric ring (624) mounted on a metal disc (626) is attached in closed proximity to the nozzle array (622) such that ultrasonic energy from the vibrator element is transferred directly to the nozzle array. The metal disc (626) may be shaped such that it may be accommodated in the curve of the venturi in an arrangement similar to that of Figure 5. The diameter of the metal disc is preferably about 20mm and it is attached over a central portion of about 4mm diameter. The vibrator element is preferably driven at high frequency e.g. 250 to 400 kHz to provide a good flow rate through the aerosol generator and to reduce the effect of bubble formation. In accordance with the invention such an aerosol generator may be sealed by a nozzle cover as shown in Figure 5.

## Claims

1. An inhalation device comprising a housing (1,344,416) containing a medicament reservoir and defining a patient port in the form of a mouthpiece (2) or nasal adaptor, means for metering a dose of medicament from said medicament reservoir and means to aerosolise said dose of medicament, the inhalation device additionally comprising a cover (7, 346, 430, 504) for the patient port or aerosolisation means movable between open and closed positions, the cover requiring an inspiration independent energy source for opening characterised in that the cover has a self-closing action (6) such that the patient port or aerosolisation means is sealed to protect the medicament whenever the inhalation device is released by the patient.

2. An inhalation device as claimed in Claim 1 in which the self-closing action of the cover is achieved by mechanical, electromechanical, pneumatic or hydraulic means.

3. An inhalation device as claimed in Claim 1 or Claim 2 comprising actuation means to initiate the self-closing action of the cover.

4. An inhalation device as claimed in Claim 3 in which the actuation means comprises a button, switch, slide or lever.

5. An inhalation device as claimed in Claim 1 or Claim 2 comprising means to detect patient inspiration through the patient port and means to initiate the self-closing action of the cover (346,430,504) when the patient ceases to inspire through the patient port after delivering a dose of medicament.

6. An inhalation device as claimed in any preceding Claim in which the cover is positioned at the end of the mouthpiece (2).

7. An inhalation device as claimed in Claim 6 in which the cover comprises a pair of flexible shutters (7) resiliently biased to the closed position and a pair of pins (4) projecting through the wall of the mouthpiece which are movable to open said shutters.

8. An inhalation device as claimed in any one of Claims 1 to 5 in which the aerosolisation means comprises an aerosol generator for generating atomised droplets (272) of liquid by cyclically pressurising a liquid in a chamber causing the liquid to be expelled through orifices of a nozzle arrangement (250) in the aerosol generator, and a cover (346,430,504) having a self-closing action to seal the nozzle arrangement (250).

9. An inhalation device as claimed in Claim 8 in which the cover is substantially cylindrical (348) having an aperture (350) extending diametrically therethrough and capable of rotation between a closed position in which the nozzle arrangement (250) is sealed and a dispensing position in which the atomised droplets from the nozzle arrangement (250) are emitted through the aperture (350) in the cover.

10. An inhalation device as claimed in Claim 8 in which the cover comprises a shutter movable in a direction parallel to the plane of the orifices of the nozzle arrangement (250).

11. An inhalation device as claimed in Claim 8 in which the cover (430) is movable between sealing and dispensing positions in a direction normal to the plane of the orifices of the nozzle arrangement (250,432).

## Patentansprüche

1. Inhalationsvorrichtung mit einem Gehäuse (1, 344, 416), das einen Medikamentenspeicher aufweist und eine Patientenöffnung in der Form eines Mundstücks (2) oder Nasenadapters festlegt, einer Einrichtung zum Bemessen einer Medikamentendosis aus dem Medikamentenspeicher und mit einer Einrichtung zum Aerosolisieren der Medikamentendosis, wobei die Inhalationsvorrichtung zusätzlich eine Abdeckung (7, 346, 430, 504) für die Patientenöffnung oder die Aerosolisiereinrichtung aufweist, die zwischen einer geöffneten und einer geschlossenen Stellung bewegbar ist, wobei die Abdeckung eine von dem Inhalieren unabhängige Energiequelle zum Öffnen erfordert, dadurch gekennzeichnet, daß die Abdeckung eine Selbstschließfunktion (6) aufweist, die so beschaffen ist, daß die Patientenöffnung oder Aerosolisiereinrichtung verschlossen ist, um das Medikament zu schützen, wenn die Inhalationsvorrichtung von dem Patienten freigegeben wird.

2. Inhalationsvorrichtung nach Anspruch 1, bei der die Selbstschließfunktion der Abdeckung durch mechanische, elektromechanische, pneumatische oder hydraulische Mittel erzielt wird.

3. Inhalationsvorrichtung nach Anspruch 1 oder 2, die eine Betätigungseinrichtung zum Auslösen der Selbstschließfunktion der Abdeckung aufweist.

4. Inhalationsvorrichtung nach Anspruch 3, bei der die Betätigungseinrichtung einen Knopf, Schalter, Schieber oder Hebel aufweist.

5. Inhalationsvorrichtung nach Anspruch 1 oder 2, die eine Einrichtung zum Detektieren des Einatmen des Patienten durch die Patientenöffnung und eine Einrichtung zum Auslösen der Selbstschließfunktion der Abdeckung (346, 430, 504), wenn der Patient damit aufhört, durch die Patientenöffnung einzuatmen, nachdem eine Medikamentendosis abgegeben wurde, aufweist.

6. Inhalationsvorrichtung nach einem der vorstehenden Ansprüche, bei der die Abdeckung an dem Ende des Mundstücks (2) angeordnet ist.

7. Inhalationsvorrichtung nach Anspruch 6, bei der die Abdeckung ein Paar flexibler Verschlußelemente (7) aufweist, die federnd zu der geschlossenen Stellung hin vorgespannt sind, sowie ein Paar Stifte (4), die durch die Wand des Mundstücks hervorstehen und zum Öffnen der Klappen bewegbar sind.

8. Inhalationsvorrichtung nach einem der Ansprüche 1 bis 5, bei der die Aerosolisiereinrichtung einen Aerosolerzeuger zum Erzeugen zerstäubter Flüssigkeitströpfchen (272) durch zyklisches Unterdrucksetzen einer Flüssigkeit in einer Kammer, das bewirkt, daß die Flüssigkeit durch Öffnungen in einer Düsenanordnung (250) in dem Aerosolerzeuger ausgestoßen wird, sowie eine Abdeckung (346, 430, 504) mit einer Selbstschließfunktion zum Verschließen der Düsenanordnung (250) aufweist.

9. Inhalationsvorrichtung nach Anspruch 8, bei der die Abdeckung im wesentlichen zylindrisch (348) ist mit einer Öffnung (350), die diametral hindurchverlauft und zwischen einer geschlossenen Stellung, in der die Düsenanordnung (250) verschlossen ist, und einer Abgabestellung, in der die zerstäubten Tröpfchen von der Düsenanordnung (250) durch die Öffnung (350) in der Abdeckung ausgeströmt werden, drehbar ist.

10. Inhalationsvorrichtung nach Anspruch 8, bei der die Abdeckung ein Verschlußelement aufweist, das in einer Richtung parallel zu der Ebene der Öffnungen der Düsenanordnung (250) bewegbar ist.

11. Inhalationsvorrichtung nach Anspruch 8, bei der die Abdeckung (430) zwischen der Schließstellung und der Abgabestellung in eine Richtung senkrecht zu der Ebene der Öffnungen der Düsenanordnung (250, 432) bewegbar ist.

## Revendications

1. Dispositif d'inhalation comportant un boîtier (1, 344, 416) contenant un réservoir de médicament et définissant un accès pour patient se présentant sous la forme d'un embout (2) ou d'un adaptateur nasal, des moyens pour mesurer une dose de médicament provenant dudit réservoir de médicament et des moyens pour transformer en un aérosol ladite dose de médicament, le dispositif d'inhalation comportant de plus un couvercle (7, 346, 430, 504) pour l'accès destiné au patient ou des moyens de transformation en aérosol mobiles entre des positions ouverte et fermée, le couvercle nécessitant une source d'énergie indépendante de l'inspiration pour s'ouvrir et ayant une action de fermeture automatique (6) de telle sorte que l'accès destiné au patient ou les moyens de transformation en aérosol soient obturés de manière étanche afin de protéger le médicament chaque fois que le dispositif d'inhalation est relâché par le patient.

2. Dispositif d'inhalation selon la revendication 1, dans lequel l'action de fermeture automatique du couvercle est obtenue par l'intermédiaire de moyens mécaniques, électromécaniques, pneumatiques ou hydrauliques.

3. Dispositif d'inhalation selon la revendication 1 ou 2 comportant des moyens d'actionnement pour amorcer l'action de fermeture automatique du couvercle.

4. Dispositif d'inhalation selon la revendication 3, dans lequel les moyens d'actionnement peuvent être un bouton, un commutateur, un élément coulissant ou un levier.

5. Dispositif d'inhalation selon la revendication 1 ou 2, comportant des moyens pour détecter une inspiration du patient à travers l'accès destiné au patient et des moyens pour amorcer l'action de fermeture automatique du couvercle (346, 430, 504) lorsque le patient cesse d'inspirer à travers l'accès destiné au patient après l'administration d'une dose de médicament.

6. Dispositif d'inhalation selon l'une quelconque des revendications précédentes, dans lequel le couvercle est positionné au niveau de l'extrémité de l'embout (2).

7. Dispositif d'inhalation selon la revendication 6, dans lequel le couvercle comporte deux obturateurs flexibles (7) rappelés de manière élastique vers la position fermée, et deux tiges (4) faisant saillie à travers la paroi de l'embout, lesquelles sont mobiles pour ouvrir lesdits obturateurs.

8. Dispositif d'inhalation selon l'une quelconque des revendications 1 à 5, dans lequel les moyens de transformation en aérosol comportent un générateur d'aérosol destiné à engendrer des gouttelettes atomisées (272) de liquide en mettant sous pression de manière cyclique un liquide contenu dans une chambre, en amenant le liquide à être expulsé à travers les orifices d'un agencement de buses (250) situé dans le générateur d'aérosol, et un couvercle (346, 430, 504) ayant une action de fermeture automatique pour obturer de manière étanche l'agencement de buses (250).

9. Dispositif d'inhalation selon la revendication 8, dans lequel le couvercle est à peu près cylindrique (348) en ayant une ouverture (350) s'étendant diamétralement à travers celui-ci et capable de tourner entre une position fermée dans laquelle l'agencement de buses (250) est obturé de manière étanche et une position d'administration dans laquelle les gouttelettes atomisées provenant de l'agencement de buses (250) sont émises à travers l'ouverture (350) formée dans le couvercle.

10. Dispositif d'inhalation selon la revendication 8, dans lequel le couvercle comporte un obturateur mobile dans une direction parallèle au plan des orifices de l'agencement de buses (250).

11. Dispositif d'inhalation selon la revendication 8, dans lequel le couvercle (430) est mobile entre des positions de fermeture et d'administration dans une direction normale au plan des orifices de l'agencement de buses (250, 432).
